# EUROPEAN PATENT APPLICATION

(11) **EP 4 494 642 A1**
(43) Date of publication of application: **22.01.2025**
(21) Application number: 23781264.9
(22) Date of filing: 27.03.2023
(51) Int. Cl.: A61K 31/5375, A61K 45/06, A61P 3/04

(54) **COMBINATION THERAPY FOR PREVENTING OR TREATING OBESITY**

(30) Priority: 28.03.2022 KR 20220038399
(71) Applicant: LG Chem, Ltd., Yeongdeungpo-gu Seoul 07336 (KR)
(72) Inventor: PARK, Hee Dong, Seoul 07796 (KR); YEO, Su Jin, Seoul 07796 (KR); HWANG, Hye Kyeong, Seoul 07796 (KR)
(74) Representative: Hoffmann Eitle
(86) International application number: PCT/KR2023/004009
(87) International publication number: WO 2023/191408

(57) **Abstract**

The present invention relates to a combination therapy for effectively preventing or treating obesity. More specifically, the objective of the present invention is to effectively prevent or treat obesity through a synergistic effect obtained when a melanocortin-4 receptor (MC4R) agonist and a glucagon-like peptide-1 (GLP-1) receptor agonist are administered in combination. 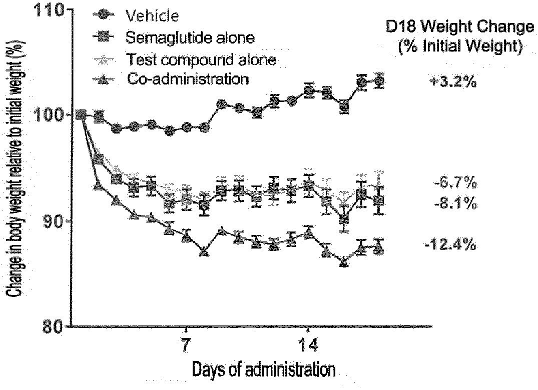 AA
Weight change rate with respect to initial weight
BB
Number of days of administration
CC
Semaglutide alone
DD
Test compound alone
EE
In combination
FF
D18 weight change rate (% initial weight)
GG
Vehicle

## Description

### Technical Field

The present invention relates to a combination therapy for efficiently preventing or treating obesity, more specifically, to efficiently preventing or treating obesity through a synergistic effect obtained from co-administration of a melanocortin-4 receptor (MC4R) agonist and a glucagon-like peptide-1 (GLP-1) receptor agonist.

### Background

Melanocortin receptor (MCR) is a type of G-protein coupled receptor (GPCR), where the primary role of G-proteins is to activate secondary messengers, thereby regulating cellular responses to a variety of physiological stimuli through signal transduction. To date, 5 types of melanocortin receptors have been identified. MC1R is expressed in melanocytes and macrophages, where it regulates melanin production in melanocytes, thereby determining the color of skin and hair. MC2R is expressed in the adrenal glands and adipose tissues and is well known for its role in mediating the secretion of adrenal hormones in response to adrenocorticotropic hormone in the adrenal glands. MC3R, MC4R, and MC5R are expressed not only in nerve terminals but also in the brain, where they are believed to mediate the effects of melanocortin peptides in the central nervous system, which influence behavior, learning, memory, appetite, and the formation and regeneration of neurons. To date, MC3R has been associated with erectile dysfunction and inflammatory responses, while MC4R has been linked to obesity and diabetes, with extensive research being conducted on the receptor-specific actions (MacNeil DJ et al., Eur J Pharmacol 2002, 450, 93). Genetic studies in individuals with advanced obesity have shown a strong involvement of MC4R, and it has been demonstrated that MC4R-knockout mice develop obesity due to hyperphagia, proving the critical role of this receptor in appetite regulation (Lu D, Willard D et al., Nature 1994, 371(6500), 799; Huszar D et al., Cell 1997, 88(1), 131; Hinney A et al., J Clin Endocrinol Metab 1990, 84(4), 1483).

The majority of currently developed anti-obesity drugs are appetite suppressants that act on the central nervous system, particularly those that regulate the activity of neurotransmitters (e.g., phentermine, mazindol, lorcaserin, fluoxetine, and sibutramine). However, these neurotransmitter modulators exert broad effects on various physiological functions beyond appetite suppression through numerous subtype receptors. As a result, these drugs lack selectivity for specific subtypes, leading to the drawback of various side effects when administered long-term. In contrast, melanocortin agonists, which are neuropeptides rather than neurotransmitters, may have an advantage with respect to a target site, as evidenced by the fact that upon administration in MC4R knockout (KO) mice, all functions other than energy metabolism remain normal, indicating that they can induce weight loss through appetite suppression without affecting other physiological functions.

Meanwhile, glucagon-like peptide-1 (GLP-1) is an incretin that plays a role in promoting the secretion of insulin from beta cells, increasing glucose utilization in peripheral tissues, inhibiting the secretion of glucagon from alpha cells, and increasing glucose production in the liver. However, endogenous GLP-1 is quickly degraded by dipeptidyl peptidase-4 (DPP-4) within a few minutes and loses its activity. GLP-1 receptor agonist is a GLP-1 analog that is not hydrolyzed by DPP-4.

### Description of the Invention

### Technical Problem

The present invention aims to provide a combination therapy capable of efficiently preventing or treating obesity that has excellent physical constitution improvement effect and safety.

### Means for Solving the Problem

To address the aforementioned problem, the present invention provides a pharmaceutical composition for preventing or treating obesity, comprising a melanocortin-4 receptor (MC4R) agonist or a pharmaceutically acceptable salt thereof, for use in combination with a glucagon-like peptide-1 (GLP-1) receptor agonist.

In addition, the present invention provides a kit for combination prevention or treatment for obesity comprising the following sets (i) and (ii):
(i) a preparation containing a glucagon-like peptide-1 (GLP-1) receptor agonist, and
(ii) a preparation containing a melanocortin-4 receptor (MC4R) agonist or a pharmaceutically acceptable salt thereof.

In addition, the present invention provides a method of preventing or treating obesity, comprising co-administering a glucagon-like peptide-1 (GLP-1) receptor agonist and a melanocortin-4 receptor (MC4R) agonist or a pharmaceutically acceptable salt thereof to a subject in need thereof in a pharmaceutically effective amount.

Hereinbelow, the present invention will be described in detail.

The present invention has shown that co-administration of a MC4R agonist and a GLP-1 receptor agonist may show, through a synergistic effect, excellent preventative or therapeutic efficacy for obesity.

According to an aspect of the present invention, provided is a pharmaceutical composition for preventing or treating obesity, comprising the compound of Formula 1 or a pharmaceutically acceptable salt thereof, for use in combination with a glucagon-like peptide-1 (GLP-1) receptor agonist:

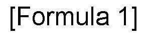

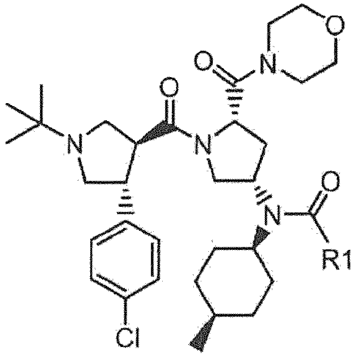

In Formula 1, R1 is C₂-C₅ alkyl.

In one example according to the present invention, the compound of Formula 1 is N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1S,4R)-4-methylcyclohexyl)isobutyramide of Formula 2:

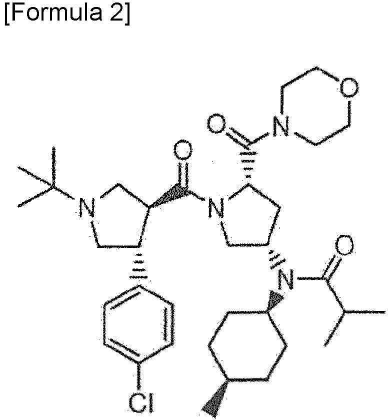

In another example according to the present invention, the pharmaceutically acceptable salt may comprise, but is not limited to, for example, inorganic acids such as hydrochloric acid, sulfuric acid, nitric acid, phosphoric acid, hydrobromic acid, and hydroiodic acid, organic carboxylic acids such as tartaric acid, formic acid, citric acid, acetic acid, trichloroacetic acid, trifluoroacetic acid, gluconic acid, benzoic acid, lactic acid, fumaric acid, and maleic acid, and sulfonic acids such as methanesulfonic acid, benzenesulfonic acid, p-toluenesulfonic acid, or naphthalenesulfonic acid. In another example according to the present invention, the pharmaceutically acceptable salt may be selected from the group consisting of hydrochloride, sulfate, nitrate, phosphate, hydrobromide, and hydroiodide. In another example according to the present invention, the pharmaceutically acceptable salt may be hydrochloride.

In another example according to the present invention, the hydrochloride salt of the compound of Formula 1 may be prepared according to Reaction Scheme 1 below. However, a person of ordinary skill in the art to which the present invention pertains may prepare the compound of Formula 1 by a variety of methods based on the structure of Formula 1.

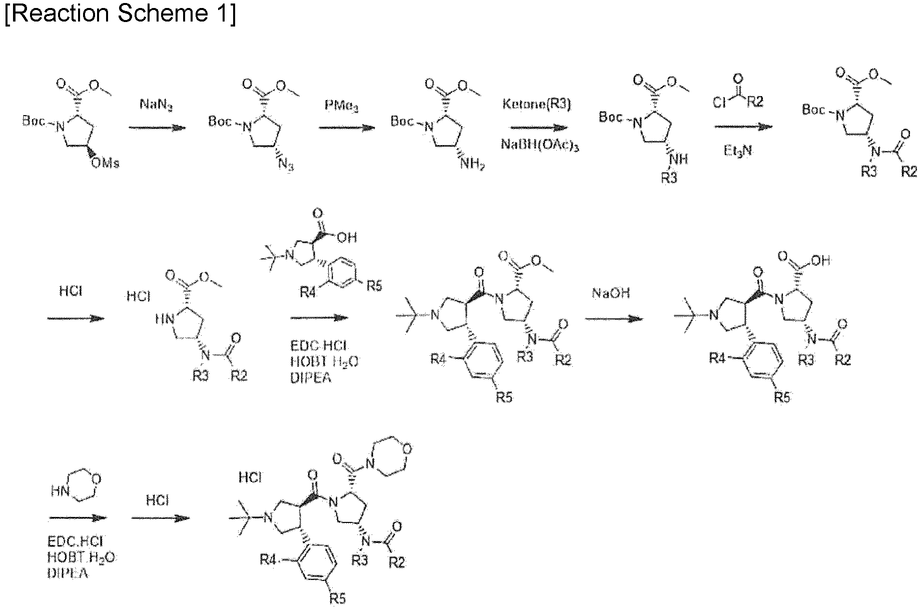

In Reaction Scheme 1 above,
R2 is C₁-C₅ alkyl;
R3 is C₃-C₈ cycloalkyl unsubstituted or substituted with one or two C₁-C₅ alkyls; and
R4 and R5 are each independently hydrogen or halogen.

In another example of the present invention, the GLP-1 receptor agonist may be a peptide drug or a small molecule. As commonly understood in the field, the small molecule refers to an organic compound with a molecular weight of approximately less than 900 Daltons, which is not a peptide drug composed of amino acids. In another example according to the present invention, the peptide drug may be selected from the group consisting of, for example, semaglutide, exenatide, liraglutide, lixisenatide, albiglutide, dulaglutide, benaglutide, PEG-loxenatide, efpeglenatide, and vurolenatide, but is not limited thereto. In another example according to the present invention, the small molecule may be danuglipron (PF-06882961) or LY-3502970, but is not limited thereto. In another example according to the present invention, the GLP-1 receptor agonist may be selected from the group consisting of semaglutide, exenatide, liraglutide, lixisenatide, albiglutide, and dulaglutide. In another example according to the present invention, the GLP-1 receptor agonist may be semaglutide or danuglipron (PF-06882961).

In another example according to the present invention, the term "therapeutically effective amount" for an individual subject refers to an amount sufficient to achieve the aforementioned pharmacological effect, i.e., the therapeutic effect. The amount of the compound may vary depending on the condition of the subject, the severity of the condition, the mode of administration, and the age of the subject to be treated. However, a person skilled in the art may determine an appropriate amount based on their own knowledge.

In another example according to the present invention, the therapeutically effective dosage of the compound of Formula 1, for example, the dosage required for treatment of an adult typically ranges from about 0.1 to 500 mg per day, depending on the frequency and intensity of administration. When administered intramuscularly or intravenously to an adult, a total daily dosage of about 0.1 to 300 mg, divided into single doses, is generally sufficient, although in some cases, a higher daily dosage may be desirable for certain patients.

In the present invention, the term "pharmaceutical composition" may comprise, in addition to the active compounds of the present invention, other chemical ingredients such as carriers, diluents, excipients, and the like. Accordingly, the pharmaceutical composition may optionally comprise pharmaceutically acceptable carriers, diluents, excipients, or combinations thereof. The pharmaceutical composition facilitates the administration of the active compounds into a biological organism. Various techniques exist for administering compounds, comprising but not limited to oral, injectable, aerosol, parenteral, and topical administration.

In the present specification, the term "carrier" refers to a compound that facilitates the delivery of a compound into cells or tissues. For example, dimethyl sulfoxide (DMSO) is a common carrier that facilitates the delivery of many organic compounds into the cells or tissues of an organism.

In the present specification, the term "diluent" is defined as a compound that not only stabilizes the biologically active form of a target compound but is also diluted in water in which the compound is dissolved. Salts dissolved in a buffer solution are used as diluents in the relevant field. Commonly used buffers include phosphate-buffered saline, which mimics the salt form of bodily fluids. Because buffer salts can control the pH of the solution at low concentrations, it is rare for buffered diluents to alter the biological activity of the compound.

In the present specification, the term "pharmaceutically acceptable" refers to properties that do not compromise the biological activity or physical properties of a compound.

The compound of the present invention may be formulated into various pharmaceutical dosage forms depending on the intended purpose. When preparing a pharmaceutical composition according to the present invention, the active ingredient, specifically a compound of Formula 1 or a pharmaceutically acceptable salt thereof, is mixed with various pharmaceutically acceptable carriers selected based on the desired dosage form. For example, a pharmaceutical composition according to the present invention may be formulated as an injectable preparation, an oral preparation, or other forms depending on the intended purpose.

The compound of the present invention may be formulated into unit dosage forms or incorporated into multi-dose containers by known methods involving known pharmaceutically acceptable carriers and excipients. The formulations may be in the form of solutions, suspensions, or emulsions in oily or aqueous media, and may contain conventional dispersing agents, suspending agents, or stabilizers. In addition, for example, the formulations may be in the form of dry powders that are reconstituted with sterile, pyrogen-free water prior to use. The compound of the present invention may also be formulated into suppositories using conventional suppository bases such as cocoa butter or other glycerides. Solid oral dosage forms may include capsules, tablets, pills, powders, and granules, with capsules and tablets being particularly preferred. Tablets and pills are preferably manufactured with enteric coatings. Solid dosage forms may be prepared by mixing the compound of the present invention with one or more inert diluents such as sucrose, lactose, or starch, and with carriers such as lubricants (e.g., magnesium stearate), disintegrants, and binders. Furthermore, the compound may be formulated for transdermal administration, and may be prepared in forms such as lotions, ointments, gels, creams, patches, or sprays. In another example of the present invention, the pharmaceutical agent or pharmaceutical composition may be in an oral dosage form.

In the present specification, the term "prevention" refers to reducing or eliminating the likelihood of developing a disease.

In the present specification, the term "treatment" refers to halting, delaying, or alleviating the progression of a disease when applied to a subject exhibiting symptoms of the disease.

According to another aspect of the present invention, provided is a kit for combination prevention or treatment for obesity, comprising the following set of (i) and (ii):
(i) a preparation containing a glucagon-like peptide-1 (GLP-1) receptor agonist, and
(ii) a preparation containing the compound of Formula 1 or a pharmaceutically acceptable salt thereof.

In another example according to the present invention, the kit allows simultaneous, sequential, or separate administration of a preparation containing a GLP-1 receptor agonist, and a preparation containing the compound of Formula 1 or a pharmaceutically acceptable salt thereof.

In another example according to the present invention, a preparation containing each of the active ingredients may further comprise a pharmaceutically acceptable excipient.

In another example according to the present invention, a kit may comprise a packaging container, an instruction manual, attached documents, and the like, in addition to a preparation containing the active ingredient. The packaging container, instruction manual, and attached documents may disclose combinations for combination use with substances. In addition, the form of combination use when administering an additional substance, or the form as a mixture, or the usage, dosage, and the like may be disclosed.

### Advantages of the Invention

According to the present invention, due to a synergistic effect obtained through a combination use of a melanocortin-4 receptor (MC4R) agonist of Formula 1 above or a pharmaceutically acceptable salt thereof, and a glucagon-like peptide-1 (GLP-1) receptor agonist, excellent physical constitution improvement effect and safety may be obtained, and obesity may be efficiently prevented or treated.

### Brief Descriptions of the Drawings

FIG. 1 is a graph illustrating the results of measuring the effect of weight loss over 18 days following co-administration of the compound prepared according to Preparation Example (hereinafter referred to as 'test compound') and semaglutide in a diet-induced obesity (DIO) mouse model.
FIG. 2 is a graph illustrating the results of measuring the effect of weight loss after 18 days following co-administration of the test compound and semaglutide in a diet-induced obesity (DIO) mouse model.
FIG. 3 is a graph illustrating the results of measuring the effect of reduction in liver and fat weight after 3 weeks following co-administration of the test compound and semaglutide in a diet-induced obesity (DIO) mouse model.
FIG. 4 is a graph illustrating the results of measuring the effect of weight loss after 18 days following co-administration of the test compound and semaglutide in a diet-induced obesity (DIO) mouse model.
FIG. 5 is a graph illustrating the results of measuring the effect of reduction in food intake over 28 days following co-administration of the test compound and PF-06882961 in a diet-induced obesity (DIO) mouse model.
FIG. 6 is a graph illustrating the results of measuring the effect of weight loss over 28 days following co-administration of the test compound and PF-06882961 in a diet-induced obesity (DIO) mouse model.
FIG. 7 is a graph illustrating the results of measuring the effect of reduction in body fat mass after 28 days following co-administration of the test compound and PF-06882961 in a diet-induced obesity (DIO) mouse model.
FIG. 8 is a graph illustrating the results of measuring the effect of improvement in glucose tolerance after 28 days following co-administration of the test compound and PF-06882961 in a diet-induced obesity (DIO) mouse model.

### Detailed Description of the Invention

Hereinafter, the present invention will be described in greater detail with reference to embodiments of the present invention. However, these embodiments are provided merely for illustrative purposes to aid in understanding the present invention, and the scope of the present invention is not limited thereto.

### Preparation Example: Synthesis of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

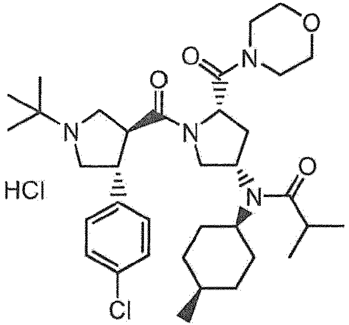

The title compound was obtained through the steps A, B, C, and D below.

### Step A: Preparation of methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate

Methyl (2S,4S)-1-((3S,4R)-1-(tert-butyl)-4-(4-chlorophenyl)pyrrolidine-3-carbonyl)-4-(*N*-((1*s*,4*R*)-4-methylcyclohexyl)isobutyramido)pyrrolidine-2-carboxylate (28.7 g, 82.73 mmol), (3S,4R)-1-(*tert*-butyl)-4-(4-chlorophenyl)pyrrolidine-3-carboxylic acid (24.5 g, 86.87 mmol), 1-(3-dimethylaminopropyl)-3-ethylcarbodiimide hydrochloride (22.2 g, 115.83 mmol), and 1-hydroxybenzotriazole hydrate (15.7 g, 115.83 mmol) were dissolved in *N,N'-*dimethylformamide (400 ml) and *N,N*'-diisopropylethylamine (72.0 ml, 413.66 mmol) was slowly added. After stirring for 16 hours at room temperature and concentrating the reaction solvent under reduced pressure, 0.5 N sodium hydroxide aqueous solution was added and extraction was performed twice with ethyl acetate. After washing the organic layer twice each with an aqueous solution of sodium chloride and water, dry filtration was performed with anhydrous magnesium sulfate. The filtrate was concentrated under reduced pressure and purified by column chromatography to obtain the title compound (41.19 g, 87%).
MS [M+H] = 575 (M+1)

### Step B: Preparation of (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylic acid

Methyl (2*S*,4*S*)-1-((3S,4R)-1-(*tert*-butyl)-4-(4-chlorophenyl)pyrrolidine-3-carbonyl)-4-(*N*-((1S,4R)-4-methylcyclohexyl)isobutyramido)pyrrolidine-2-carboxylate obtained in Step A (39.4 g, 68.62 mmol) was dissolved in methanol (450 ml) and 6N sodium hydroxide aqueous solution (57.2 ml, 343.09 mmol) was added. After stirred for 16 hours at room temperature and adjusted to about pH 5 with 6N hydrochloric acid aqueous solution, the reaction solution was concentrated under reduced pressure. After dissolving the concentrated solution with dichloromethane, the undissolved solids were filtered with a paper filter. The filtrate was concentrated under reduced pressure to obtain a crude product (38.4 g, 99%) which without purification was used in the subsequent step.
MS [M+H] = 561 (M+1)

### Step C: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide

(2*S*,4*S*)-1-((3*S*,4*R*)-1-(*tert*-butyl)-4-(4-chlorophenyl)pyrrolidine-3-carbonyl)-4-(*N-*((1*s*,4*R*)-4-methylcyclohexyl)isobutyramido)pyrrolidine-2-carboxylic acid obtained in Step B (38.4 g, 68.60 mmol), 1-(3-dimethylaminopropyl)-3-ethylcarbodiimide hydrochloride (18.4 g, 96.04 mmol), and 1-hydroxybenzotriazole hydrate (13.0 g, 96.04 mmol) were dissolved in N, N'-dimethylformamide (200 ml), and morpholine (5.9 ml, 68.80 mmol) and N,N'-diisopropylethylamine (59.7 ml, 343.02 mmol) were slowly and sequentially added. After stirring for 16 hours at room temperature and concentrating the reaction solution under reduced pressure, 0.5 N aqueous solution of sodium hydroxide was added and extraction was performed twice with ethyl acetate. After washing the organic layer twice each with a sodium chloride aqueous solution and water, dry filtration was performed with anhydrous magnesium sulfate. The filtrate was concentrated under reduced pressure and purified by column chromatography to obtain the title compound (37.05 g, 86%). MS [M+H] = 630 (M+1)

### Step D: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

*N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-N-((1S,4R)-4-methylcyclohexyl)isobutyramide obtained in Step C (5.0 g, 7.95 mmol) was dissolved in ethyl acetate (50 ml) and 2N hydrochloric acid ethyl acetate solution (3.97 ml, 15.89 mmol) was slowly added. After stirring for 30 minutes at room temperature, the reaction solvent was concentrated under reduced pressure. The produced crude solids were purified by trituration using hexane and diethyl ether to obtain the title compound (5.23 g, 99%).
MS [M+H] = 630 (M+1)
¹H NMR (500 MHz, CD ₃OD) δ 7.49-7.44 (m, 4H), 4.83 (m, 1H), 4.23-4.20 (m, 1H), 3.95-3.91 (m, 2H), 3.79-3.47 (m, 14H), 3.03-3.00 (m, 1H), 2.86-2.82 (m, 1H), 2.73-2.67 (m, 1H), 2.20-2.14 (m, 1H), 1.97 (m, 1H), 1.80-1.62 (m, 5H), 1.50 (s, 9H), 1.44-1.27 (m, 3H), 1.06-1.04 (m, 9H)

### Example 1: Measurement of Weight Loss Effect

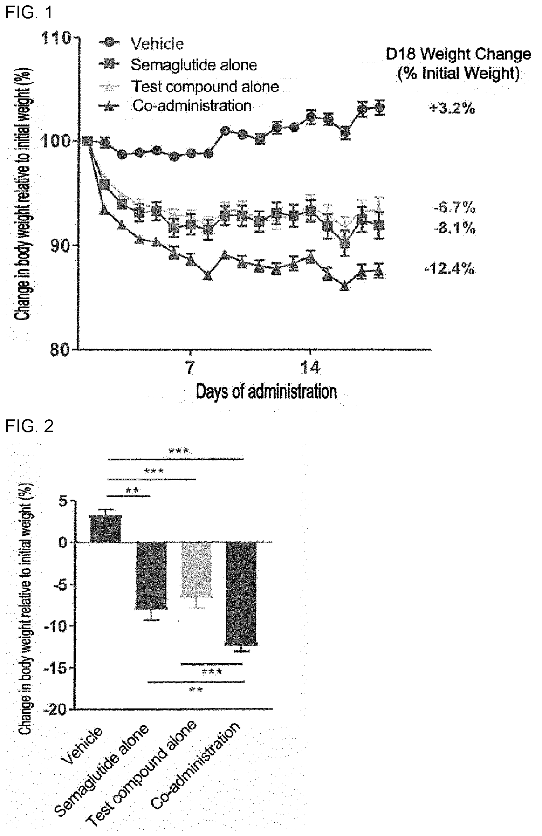

In a diet-induced obesity (DIO) mouse model where obesity was induced using a high-fat diet, the weight loss effect was measured over 18 days following repeated co-administration of semaglutide (a GLP-1 receptor agonist, trade name: Ozempic) and an MC4R agonist, N-((3S,5S)-1-((3S,4R)-1-(tert-butyl)-4-(4-chlorophenyl)pyrrolidine-3-carbonyl)-5-(morpholin-4-carbonyl)pyrrolidin-3-yl)-N-((1S,4R)-4-methylcyclohexyl)isobutyramide hydrochloride obtained in Preparation Example (hereinafter referred to as 'test compound'). Body weight measurements were taken once daily prior to the administration of the test substances, and changes in body weight compared to weight (D1) prior to the administration of the test substances were calculated and presented in FIGS. 1 and 2. By comparing the weight loss effect in the co-administration group to that in the single administration group, the effect of co-administration of a GLP-1 receptor agonist and the test compound was evaluated. As shown in FIGS. 1 and 2, the co-administration of semaglutide, which is a GLP-1 receptor agonist, and the test compound demonstrated a synergistic weight loss effect.

### Example 2: Measurement of the Effect of Reduction in Liver/Fat Weight

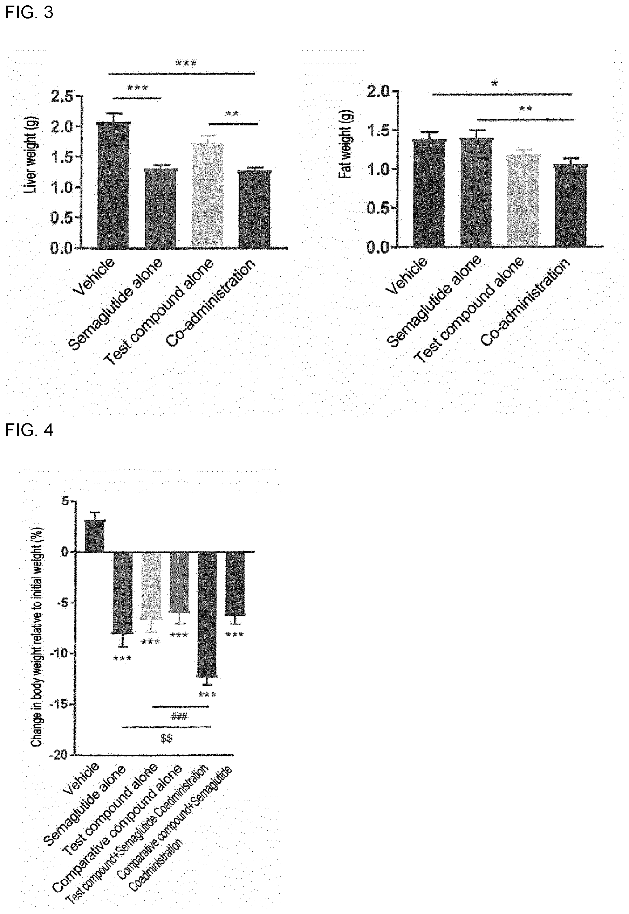

In a diet-induced obesity (DIO) mouse model where obesity was induced using a high-fat diet, the effect of reduction in liver and fat weights was measured after 3 weeks of repeated co-administration of semaglutide (a GLP-1 receptor agonist, trade name: Ozempic) and the test compound, which is an MC4R agonist, with the results shown in FIG. 3. As can be seen from FIG. 3, the co-administration of semaglutide, which is a GLP-1 receptor agonist, and the test compound demonstrated a synergistic effect in reducing liver and fat weights.

### Comparative Example

In a diet-induced obesity (DIO) mouse model where obesity was induced using a high-fat diet, the weight loss effect was compared after 18 days of repeated co-administration of semaglutide (a GLP-1 receptor agonist, trade name: Ozempic) with either the test compound, which is an MC4R agonist, or setmelanotide (RM-493), which is a peptide MC4R agonist (hereinafter referred to as 'comparative compound'). Dosages of the test compound and the comparative compound were selected based on the efficacious doses observed in single administration studies (test compound: 15 mg/kg/day, comparative compound: 3 mg/kg/day). Body weight measurements were taken once daily prior to the administration of the test substances, and changes in body weight after 18 days relative to weight (D1) prior to the administration of the test substances were calculated and presented in FIG. 4. In terms of the effect due to co-administration with the GLP-1 receptor agonist, as shown in FIG. 4, the test compound demonstrated a significant synergistic weight loss effect in the co-administration group, whereas no such synergistic weight loss effect was observed in the co-administration group with the comparative compound.

### Example 3: Combination Study with PF-06882961 (danuglipron)

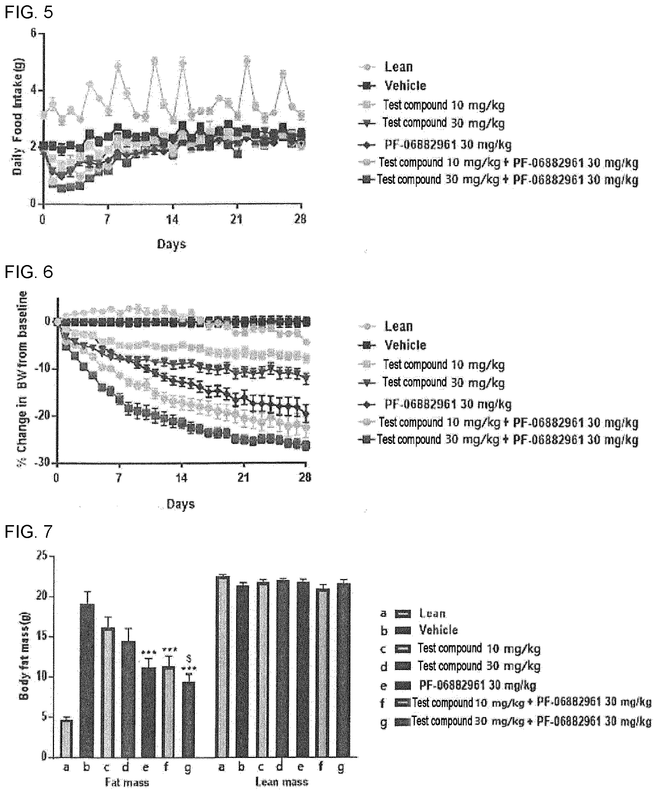

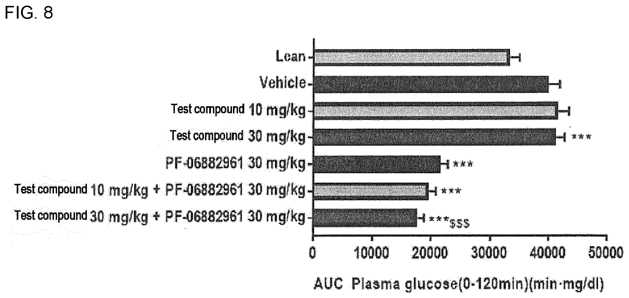

In a diet-induced obesity (DIO) mouse model where obesity was induced using a high-fat diet, changes in food intake and body weight were measured over 4 weeks of repeated co-administration of PF-06882961 (danuglipron), which is an oral GLP-1 receptor agonist, and the test compound, which is an MC4R agonist. Food intake and body weight were measured once daily prior to the administration of the test substances, and changes in body weight relative to weight (D1) prior to the administration of the test substances were calculated, with the results presented in FIGS. 5 and 6, respectively. Further, on the 28th day after the administration of the test substances, body fat mass and an oral glucose tolerance test (OGTT) were measured, with the results shown in FIGS. 7 and 8, respectively. Body fat mass was measured using a ¹H-Mini-spec system (LF-90II, Bruker Optics GmbH, Germany) and blood glucose was measured using GM9 (Analox Instruments Ltd., UK) with blood samples collected from the tail vein.

As can be seen from FIGS. 5 and 6, the co-administration of GLP-1 receptor agonist PF-06882961 and the test compound demonstrated a synergistic weight loss effect proportional to the significant reduction in food intake compared to their respective single administrations. Furthermore, as shown in FIGS. 7 and 8, it was confirmed that the combination therapy results in a much greater reduction in body fat mass and improvement in glucose tolerance compared to single administration.

## Claims

1. A pharmaceutical composition for preventing or treating obesity, for use in combination with a glucagon-like peptide-1 (GLP-1) receptor agonist, the pharmaceutical composition comprising a compound of Formula 1 or a pharmaceutically acceptable salt thereof: 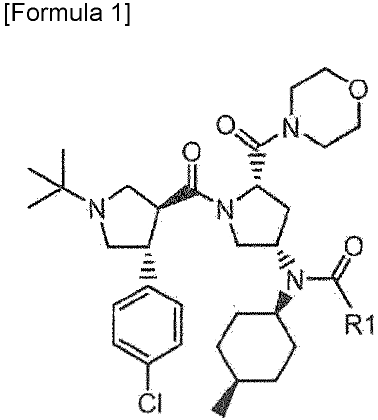 wherein in Formula 1, R1 is C₂-C₅ alkyl.

2. The pharmaceutical composition for preventing or treating obesity of claim 1, **characterized in that** the compound of Formula 1 is N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-*N*-((1S,4R)-4-methylcyclohexyl)isobutyramide of Formula 2:

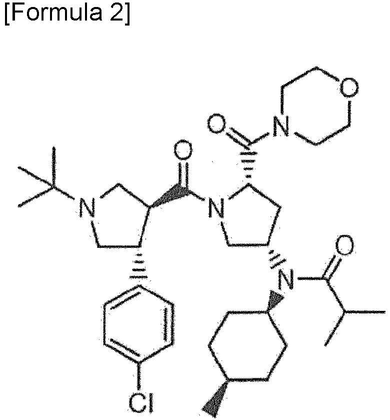

3. The pharmaceutical composition for preventing or treating obesity of claim 1, **characterized in that** the pharmaceutically acceptable salt is selected from the group consisting of hydrochlorides, sulfates, nitrates, phosphates, hydrobromides, and hydroiodates.

4. The pharmaceutical composition for preventing or treating obesity of claim 1, **characterized in that** the GLP-1 receptor agonist is a peptide drug or a small molecule.

5. A kit for combination prevention or treatment for obesity, comprising the following set of (i) and (ii):
(i) a preparation containing a glucagon-like peptide-1 (GLP-1) receptor agonist, and
(ii) a preparation containing a compound of Formula 1 or a pharmaceutically acceptable salt thereof: 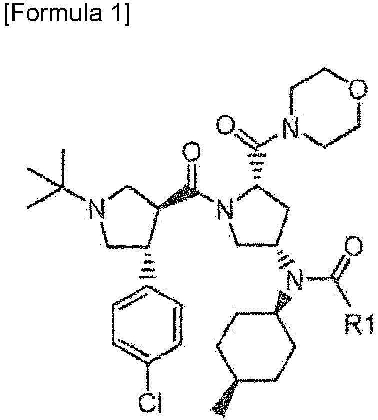
wherein in Formula 1, R1 is C₂-C₅ alkyl.

6. The kit for combination prevention or treatment for obesity of claim 5, **characterized in that** the compound of Formula 1 is N-((3S,5S)-1-((3S,4R)-1-(tert-butyl)-4-(4-chlorophenyl) pyrrolidine-3-carbonyl)-5-(morpholine-4-carbonyl)pyrrolidin-3-yl)-*N*-((1S,4R)-4-methylcyclohexyl)isobutyramide of Formula 2:

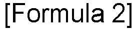

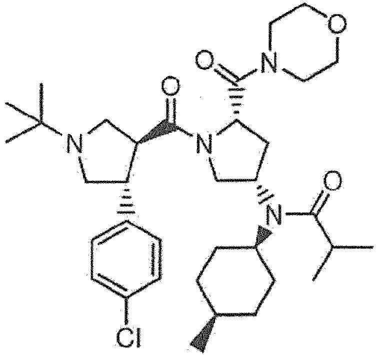

7. The kit for combination prevention or treatment for obesity of claim 5, **characterized in that** the pharmaceutically acceptable salt is selected from the group consisting of hydrochlorides, sulfates, nitrates, phosphates, hydrobromides, and hydroiodates.

8. The kit for combination prevention or treatment for obesity of claim 5, **characterized in that** the GLP-1 receptor agonist is a peptide drug or a small molecule.

9. The kit for combination prevention or treatment for obesity of claim 5, **characterized in that** a preparation containing a GLP-1 receptor agonist, and a preparation containing the compound of Formula 1 or a pharmaceutically acceptable salt thereof are simultaneously, sequentially, or separately administered.
